# EUROPEAN PATENT SPECIFICATION

(11) **EP 1 687 413 B1**
(45) Date of publication and mention of the grant of the patent: **05.03.2008**
(21) Application number: 03754305.5
(22) Date of filing: 29.10.2003
(51) Int. Cl.: C12N 5/02, C12N 5/08

(54) **METHOD FOR DIFFERENTIATING MESENCHYMAL STEM CELL INTO NEURAL CELL AND PHARMACEUTICAL COMPOSITION CONTAINING THE NEURAL CELL FOR NEURODEGENERATIVE DISEASE**
VERFAHREN ZUR DIFFERENZIERUNG EINER MESENCHYM-STAMMZELLE ZU EINER NERVENZELLE SOWIE DIE NERVENZELLE ENTHALTENDE PHARMAZEUTISCHE ZUSAMMENSETZUNG GEGEN EINE NEURODEGENERATIVE KRANKHEIT
PROCEDE PERMETTANT DE DIFFERENTIER UNE CELLULE SOUCHE MESENCHYMATEUSE DANS UNE CELLULE NERVEUSE ET COMPOSITION PHARMACEUTIQUE CONTENANT LA CELLULE NERVEUSE POUR LES MALADIES NEURODEGENERESCENTES

(43) Date of publication of application: 09.08.2006
(73) Proprietor: FCB Pharmicell Co., Ltd, Kyunggi-do 462-120 (KR); Kim, Hyun Soo, Suwon-si, Kyunggi-do 442-470 (KR)
(72) Inventor: KIM, Hyun-Soo, Youngtong-dong, Youngtong-gu, Suwon-si, Kyunggy-do 443-737 (KR); KANG, Young-Mo, Suwon-si, 441-829 Kyunggi-do (KR); LEE, Kyung-Bock, Suwon-si, 442-373 Kyunggi-do (KR); PARK, Sang-Kyo, Paldal-gu, Suwon-si, 442-733 Kyunggi-do (KR); LEE, Sang-Kap, 706-766 Daegu-si (KR)
(74) Representative: Hiebl, Inge Elisabeth
(86) International application number: PCT/KR2003/002302
(87) International publication number: WO 2005/040362

(56) References cited:
- WO-A1-96/15226
- WO-A1-02/086108
- US-A- 5 942 225
- US-B1- 6 214 334
- TAO HELEN ET AL: "Transdifferentiation of Human Haemopoietic Lineage Negative Bone Marrow Cells to Neural Cells by Cytokines and Chemical Inducing Agents." BLOOD, vol. 100, no. 11, 16 November 2002 (2002-11-16), page Abstract No. 4123, XP009073026 & 44TH ANNUAL MEETING OF THE AMERICAN SOCIETY OF HEMATOLOGY; PHILADELPHIA, PA, USA; DECEMBER 06-10, 2002 ISSN: 0006-4971
- OH Y S ET AL: "DIFFERENTIATION OF ADULT BONE MARROW STEM CELLS INTO NEURON-LIKE CELLS" ABSTRACTS OF THE SOCIETY FOR NEUROSCIENCE, SOCIETY FOR NEUROSCIENCE, WASHINGTON, DC, US, vol. 27, no. 1, 2001, page 58, XP009054180 ISSN: 0190-5295
- KOICHI I ET AL: "HUMAN PLACENTA-DERIVED MESENCHYMAL STEM CELLS DIFFERENTIATE INTO NEURAL CELLS" BLOOD, W.B.SAUNDERS COMPANY, ORLANDO, FL, US, vol. 100, no. 11, 16 November 2002 (2002-11-16), page ABSTRNO2021, XP008061487 ISSN: 0006-4971
- REYES M ET AL: "TURNING MARROW INTO BRAIN: GENERATION OF GLIAL AND NEURONAL CELLS FROM ADULT BONE MARROW MESENCHYMAL STEM CELLS" BLOOD, W.B.SAUNDERS COMPANY, ORLANDO, FL, US, vol. 94, no. 10,SUPP01PART01, 15 November 1999 (1999-11-15), page 377A, XP009007196 ISSN: 0006-4971
- SANCHEZ-RAMOS J. ET AL.: 'Adult bone marrow stromal cells differentiate into neural cells in vitro' EXP. NEUROL. vol. 164, no. 2, August 2000, pages 247 - 256, XP002199139
- SCHULDINER M. ET AL.: 'Effects of eight growth factors on the differentiation of cells derived from human embryonic stem cells' PROC. NATL. ACAD. SCI. USA vol. 97, no. 21, 10 October 2000, pages 11307 - 11312, XP002184277
- SUGAYA K. ET AL.: 'Neuroplacement therapy and stem cell biology under disease conditions' CELL. MOL. LIFE SCI. vol. 60, no. 9, September 2003, pages 1891 - 1902, XP008071706

## Description

### Technical Field

The present invention relates to a method of differentiating a mesenchymal stem cell into a neural cell. More particularly, the present invention relates to a method of differentiating a mesenchymal stem cell into a neural cell by culturing in a medium comprising an epidermal growth factor and a hepatocyte growth factor after confluent culturing the mesenchymal stem cell as a pretreatment.

### Background Art

Since a mesenchymal stem cell recently has been isolated successfully from a human, a clinical application of the mesenchymal stem cell has been brought to a focus. Particularly, in the clinical application, the mesenchymal stem cell is used as cell donor in cell replacement therapy. The cell replacement therapy is presented for effectively treating cell deficiency caused by neurodegenerative diseases such as Parkinson's disease and Alzheimer's disease, known as incurable diseases, ischemic and hemorrhagic stroke, traumatic disease and spinal cord injury, which are caused by destruction and permanent functional disorder of cells in tissues.

However, cell replacement therapy has a limitation in practical application. That is, in a conventional method of implanting cells fully differentiated into donor tissues into patients it is difficult to obtain sufficient amount of the cells to be given to the patients.

In order to solve the above-mentioned problems, differentiating the mesenchymal stem cell into a tissue-specific cell or inducing differentiation after isolating and proliferating the tissue-specific stem cell may be used in the cell replacement therapy.

Implanting a neural cell differentiated from a mesenchymal stem cell of one human into another human, however, is difficult in practice, and creates immunological reactions in case of implanting the neural cell between male and female cell donors.

Until now, differentiation of a mesenchymal stem cell of a rat into a hematopoietic cell, a myocardial cell, an islet of Langerhans and a neural cell have been proved in vitro. Studies such as a study on the implantation of an oligodendrocyte differentiated from a mesenchymal stem cell into a rat and increasing it to create myelin (Brüstle et al., Science 285:754-756), a study on the implantation of an insulin secreting cell, differentiated from a mesenchymal stem cell, into a diabetic rat and regulation of the blood sugar level (Soria et al., Diabetes 49:11157-162, 2000), a study on the implantation of a neural cell, differentiated from a mesenchymal stem cell, into a spinal cord injured rat wherein the improvement of motor disturbance in the spinal cord injured was confirmed (McDonald et al., Nat. Med. 5(12):1410-1412, 1999), etc., represent methods of implanting cells that are differentiated from mesenchymal stem cells, for effectively treating diseases due to deficiency of cells.

However, isolation of mesenchymal stem cells has been accomplished just recently and differentiation of mesenchymal stem cells into other cells except neural cells in vitro has yet to be reported, such that the clinical application of tissue-specific cells differentiated from mesenchymal stem cells for the cell replacement therapy is possible, but is not practical.

Although a method of differentiating cells from mesenchymal stem cells for cell replacement therapy is thought to be the most effective method until now, a risk from other mixed cells, due to low efficiency of the differentiation from the mesenchymal cells into the tissue-specific cells, still exists, causing immunological reactions when implanted into a patient. Therefore, studies on differentiation are required for safe clinical application.

A method of using tissue-specific stem cells for the cell replacement therapy also has problems such as differentiation into undesired cells due to modification of differentiation potential when the tissue-specific stem cell is cultured for a long time and decrease of the proliferation rate of cells. Moreover, neural cells for treating neurodegenerative disease such as Parkinson's disease require implantation. Considering that neural cells are obtained mainly by differentiating and proliferating neural stem cells from fetal brain since it is difficult to obtain neural cells directly from patients, implanting neural cells is a disadvantage. About two fetal brains are required to treat one patient in such a case as above. Implanting the neural cells has problems such as insufficient supply of the neural cells, an unethical act, differentiation of the neural stem cells into astrocytes instead of the neural cells, and generation of an immunological reaction.

Accordingly, if a method of treatment using a neural cell differentiated from a mesenchymal stem cell is possible, problems such as difficulty in obtaining sufficient cells and generation of immunological reaction may be solved. Whether a mesenchymal stem cell as a mesoderm may be differentiated into a neural cell is still in question, but trans-differentiation was recently reported and the possibility of differentiation of the mesenchymal stem cell is increased.

Formerly, hepatocytes were known to be differentiated into only specific class of a tissue cell. It was reported that mesenchymal stem cells formed in vitro colonies in a medium comprising growth factors such as basic fibroblast growth factor, transforming growth factor, epidermal growth factor, etc. (Kuznetsov et al., Br. J. Haematol. 97:561, 1997; Van den Bos C et al., Human Cell 10:45, 1997). In addition, about a third of the first anchored cells with multi-differentiative potential were differentiated into desmoplastic cells such as osteoblasts, chondroblasts, adipocytes etc. (Pttenger MF et al., Science 279:1528, 1998), and bone marrow was a resource of a myogenic precursor cell for forming new muscles (Ferrari G et al., Science 279:1528, 1998).

However, according to recent consecutive studies, it was reported that mesenchymal stem cells might be differentiated into not only desmoplastic cells, but also into neural cells. For example, it was reported that mesenchymal stem cells were differentiated into neural cells and astrocytes by culturing in medium comprising retinoic acid and BDNF (brain-derived neurotrophic factor) (Sanchez-Ramos et al., Exp. Neurology 164:247-256, 2000) and into neural cells by culturing in medium comprising antioxidative substance such as mercaptoethanol and DMSO (dimethyl sulfoxide) (Date Woodbury et al., J. Neuro. Res. 61:364-370, 2000).

When a chemical reagent such as DMSO is used to induce differentiation, however, toxicity of DMSO may alter the mesenchymal stem cells such that a safer method is required.

The inventors of the present invention studied a safe and effective method of differentiating of a mesenchymal stem cell into a neural cell, and applied for a patent relating to a "method of differentiating a mesenchymal stem cell into a neural cell" in Korean patent number 2001-21064. The patent relates to a method of differentiating and proliferating a mesenchymal stem cell into a neural cell by culturing in a medium comprising an epidermal growth factor and a hepatocyte growth factor.

The method of differentiating and proliferating a mesenchymal stem cell into a neural cell has problems not only with low rate but also about 80% efficiency of differentiation. Further, culturing for four weeks is required due to low differentiation rate when a cell during differentiation and proliferation is cultured such that morphological alteration occurs. Thus, problems, such as contamination and a large consumption of reagents, equipments and time, are generated.

The method in Korean patent number 2001-21064 is proposing the possibility that a neural cell that is differentiated from a mesenchymal stem cell is useful for treating neurodegenerative diseases such as Parkinson's disease, Alzheimer, Pick's disease, Huntington's disease, amyotrophic lateral sclerosis, and ischemic and hemorrhagic brain disease, however, in vitro tests, animal tests or clinical tests have yet to be performed and thus the pharmacological effect of the neural cells also has yet to be confirmed.

To solve the problems, the inventors completed the present invention by studying a method of improving differentiation rate and efficiency, and a test of pharmacological effect.

### Disclosure of The Invention

### Technical problem

Accordingly, it is an object of the present invention to provide a method of differentiating and proliferating a mesenchymal stem cell into a neural cell by culturing in a medium comprising an epidermal growth factor and a hepatocyte growth factor, said mesenchymal stem cell being cultured in the medium after confluent culturing of said mesenchymal stem cell.

A pharmaceutical composition comprising a neural cell for treating neurodegenerative disease such as Parkinson's disease, Alzheimer's disease, Pick's disease, Huntington's disease, amyotrophic lateral sclerosis, ischemic and hemorrhagic brain disease, and traumatic central nervous system disease such as spinal cord injury is also described.

### Technical solution

Hereinafter, the present invention is described in detail.

The present invention provides a method of differentiating a mesenchymal stem cell into a neural cell by confluent culturing in a medium comprising an epidermal growth factor and a hepatocyte growth factor.

The mesenchymal stem cells are usually cultured in state of maintaining the rate of cells that take the surface of the medium at about 70% by count for continuous growth. However, the mesenchymal stem cells in the method of the present invention are confluently cultured continuously after the cells have completely taken the surface of the medium, which is object to induce differentiation instead of proliferation. Therefore, the differentiation may be carried out rapidly and effectively when stimulation of differentiation is applied after confluent culturing according to the method of the present invention.

Preferably, the mesenchymal stem cell is confluently cultured for about 1 to about 50 hours, and is then cultured respectively in a medium comprising an epidermal growth factor at about 1 to about 10,000ng/ml and a hepatocyte growth factor at about 1 to about 10,000ng/ml for 1 week or more.

More preferably, the mesenchymal stem cell is confluently cultured for about 24 hours, and then is cultured respectively in a medium comprising an epidermal growth factor at about 10ng/ml and a hepatocyte growth factor at about 10ng/ml for 1 week or more.

When the mesenchymal stem cell is cultured as in the above-mentioned conditions, a few mesenchymal stem cells form a neural cell colony after about 4 days and the neural cell colony proliferates continuously such that a large quantity of neural cells may be formed after about 1 week.

In particular, the neural cell that is fully differentiated and proliferated in the medium comprising the epidermal growth factor and the hepatocyte growth factor for about 2 weeks is proliferated continuously without altering the character of the neural cell in a medium comprising only the epidermal growth factor.

However, a neural cell differentiated fully for about 2 weeks, differentiates by culturing in a medium comprising only the hepatocyte growth factor such that the neural cell count is decreased due to progression of only the differentiation of the neural cell. Therefore, culturing the neural cell that is differentiated fully, in the medium comprising only the epidermal growth factor is preferable after about 2 weeks.

The mesenchymal stem cell in the present invention may be obtained by isolation from human bone marrow. A hematopoietic stem cell is differentiated into a blood cell when a mononuclear cell is isolated from bone marrow, such that the mesenchymal stem cell is obtained by a method of isolating only the stem cell as an unlimited proliferating cell that remains.

However, culturing total mononuclear cells isolated from bone marrow as the above-mentioned method without isolation of the mesenchymal stem cells from the mononuclear cells shows the same conclusion - production of a large quantity of neural cells - such that using only the mesenchymal stem cells is not required.

As mentioned above, when the mesenchymal stem cells are cultured in a medium comprising an epidermal growth factor and a hepatocyte growth factor, about 90% by count of the mesenchymal stem cells are differentiated into neural cells, about 70% by count of the neural cells are neurons, about 30% by count are the astrocytes, and none are microglial cells.

In the present invention, the neural cells includes neurons, astrocytes and microglial cells.

The neural cells that are differentiated from mesenchymal stem cells by the above-mentioned method of the present invention may be used as a pharmaceutical composition for treating neurodegenerative disease in the cell replacement therapy. Accordingly, the present invention describes a pharmaceutical composition comprising the neural cells, prepared by the method of the present invention, for treating neurodegenerative diseases such as Parkinson's disease, Alzheimer, Pick's disease, Huntington's disease, amyotrophic lateral sclerosis, ischemic and hemorrhagic brain disease, traumatic central nervous system disease, and motor disturbance by injury the vertebra.

The pharmaceutical composition comprising the neural cells for treating neurodegenerative disease may be administered by formulating a unit dosage suitable for administrating to human by conventional methods in pharmaceutical field. Injection as non-oral administration is preferred.

In addition to the neural cells of the pharmaceutical composition, the above formulation may contain one or more inactivated carriers that are permitted pharmaceutically. Examples of the inactivated carriers include preservative, analgesic controller, solubilizer, stabilizer, etc., as an injection application, and gasifier, bulking agent, lubricant, stabilizer, etc., as a topical application.

The pharmaceutical formulation may be used for non-oral administration, for example intravenous, subcutaneous, intra-peritoneal administration or topical application.

For example, a clinical method described by Douglas Kondziolka (Pittsburgh, 1998) may be used. The method includes cutting a patient's skull at about 1cm diameter (of pea size) and injecting the neural cell solution mixed with Hank's balanced salt solution (HBSS) into 3 places. In a method of injecting the neural cell solution, a syringe with long needle and stereotactic frame are used for an injection. The neural cell may be injected directly or through vein and artery.

Preferably, the dosage of the neural cells is about 1x10⁶ to about 1x10⁹ cells, and may vary in accordance with the kind of disease, the degree of seriousness of disease, dosage route, weight, age and sex of patient.

As mentioned above, it was known that mesenchymal stem cells have been differentiated into neural cells by culturing in a medium comprising an epidermal growth factor and a hepatocyte growth factor in the prior inventions. However, it became known for the first time that culturing according to the present invention in a medium comprising an epidermal growth factor and a hepatocyte growth factor after confluent culture as pre-treatment may improve differentiation speed within one week, differentiation rate and maturity of the neural cell.

The present invention compared each amount of the mononuclear cells isolated from the bone marrow to be proliferated and differentiated into the neural cell in case of culturing in a medium comprising an epidermal growth factor and a hepatocyte growth factor after confluent culture for about 24 hours, and in case of culturing in a medium comprising one of the epidermal growth factor and the hepatocyte growth factor without confluent culture, respectively. As a result, in case of culturing in the medium comprising the epidermal growth factor and the hepatocyte growth factor after confluent culture for about 24 hours, the neural cell colony began to form after about 1 week and continued to proliferate even after about 2 weeks. The mononuclear cells were not differentiated into neural cells in case of culture in the medium comprising only the epidermal growth factor, and were differentiated early and proliferated in case of culturing in the medium comprising only the hepatocyte growth factor.

Accordingly, when the mesenchymal stem cells or the mononuclear cells derived from the bone marrow are differentiated and proliferated into the neural cells, the epidermal growth factor inducing proliferation, the hepatocyte growth factor inducing differentiation, and confluent culture for about 24 hours stimulates the fast effect of the growth factors. A sufficient amount of the neural cells may not be obtained from only one of the growth factors.

In the present invention, the mesenchymal stem cells or the mononuclear cells derived from the bone marrow are cultured in a medium comprising an epidermal growth factor and a hepatocyte growth factor for about 2 weeks after confluent culture for about 24 hours, and then the differentiated and proliferated cells are separated as single cells and observed with an optical microscope. As a result, neurons with long axons and short dendrites, and astrocytes with only short dendrites may be observed.

By performing an immunocytochemical staining of the differentiated and proliferated cells produced by the method according to the present invention, a neuron marker such as NeuN, NSE and MAP-2, and an astrocyte marker such as GFAP are stained positively, which may confirm that the differentiated and proliferated cells consist of neurons and astrocytes. The microglial cell marker such as OX-42 is stained negatively, which may confirm that the cells are not differentiated into microglial cells.

When the mesenchymal stem cells or the mononuclear cells derived from the bone marrow are confluently cultured in a medium comprising an epidermal growth factor and a hepatocyte growth factor for about 24 hours, after about 2 weeks, about 90% by count of cells are differentiated into neural cells, about 70% by count of the neural cells are neurons, about 30% by count are astrocytes. After the mesenchymal stem cells or the mononuclear cells are fully differentiated and proliferated after about 2 weeks, the mesenchymal stem cells or the mononuclear cells maintain the shape of neural cells and proliferate continuously in the medium comprising only the epidermal growth factor. In the medium comprising only the hepatocyte growth factor, the mesenchymal stem cells or the mononuclear cells are merely differentiated, so that the mesenchymal stem cells or the mononuclear cells do not continuously proliferate.

The mesenchymal stem cells are only isolated from the mononuclear cells so that it is confirmed whether the neural cells, derived from the mononuclear cells in the bone marrow by culturing in the medium comprising the epidermal growth factor and the hepatocyte growth factor, are differentiated from the mesenchymal stem cells. The stem cells are classified into the hematopoietic stem cells and the mesenchymal stem cells derived from the mononuclear cells in the bone marrow. The hematopoietic stem cells are easily differentiated into blood cells under general culture conditions, such that the stem cells that are continuously proliferated after about 1 to about 2 weeks may be the mesenchymal stem cells. After isolating only the mesenchymal stem cells that may be subcultured more than 20 times, the experiment of differentiation into various connective tissues is carried out so that the experiment may confirm that the mesenchymal stem cells have the ability of differentiation into various connective tissue cells. A result, the experiment has confirmed that the mesenchymal stem cells may differentiate into various connective tissue cells.

Further, it is confirmed that the mesenchymal stem cells are differentiated into neural cells and astrocytes, and are proliferated in a medium comprising an epidermal growth factor and a hepatocyte growth factor by the experiment of differentiation and proliferation into neural cells, optical microscopy and immunocytochemistry like the bone marrow-derived mononuclear cells.

### Brief Description of the Drawing

FIG. 1 shows a photograph of an anchored cell when a bone marrow-derived mononuclear cell is cultured in a medium comprising about 10ng/ml of an epidermal growth factor and about 20ng/ml of a hepatocyte growth factor, respectively, for about 1 week after confluent culture for about 24 hours, through an optical microscope (x100).
FIG. 2 shows a photograph of a neural cell when the bone marrow-derived mononuclear cell is cultured in a medium comprising about 10ng/ml of an epidermal growth factor and about 20ng/ml of a hepatocyte growth factor, respectively, for about 2 weeks after confluent culture for about 24 hours, through an optical microscope (x100).
FIG. 3 shows a photograph of the differentiated and isolated neural cell from the bone marrow-derived mononuclear cell in FIG. 2, through an optical microscope; FIG. 3a shows a neuron and FIG. 3b shows an astrocyte.
FIG. 4 shows a photograph of the differentiated neural cell from the bone marrow-derived mononuclear cell in FIG. 2 by immunocytochemistry; FIG. 4a shows a cell positively stained in NSE, FIG. 4b shows staining in NeuN, FIG. 4c shows staining in GFAP, and FIG. 4d shows staining in MAP-2.
FIG. 5 shows a photograph of the differentiated cells when the bone marrow-derived mononuclear cells are cultured in a medium comprising a low concentration of glucose and the mesenchymal stem cells are isolated and differentiated into osteoblasts (FIG. 5a), chondroblasts (FIG. 5b) and fat cells (FIG. 5c), through an optical microscope.
FIG. 6 shows a photograph of a mesenchymal stem cell when the mesenchymal stem cell is cultured in a medium comprising about 10ng/ml of an epidermal growth factor and about 20ng/ml of a hepatocyte growth factor, respectively, for about 1 week, through an optical microscope.
FIG. 7 shows a photograph of the proliferated and differentiated neural cell when the mesenchymal stem cell is cultured in a medium comprising about 10ng/ml of an epidermal growth factor and about 20ng/ml of a hepatocyte growth factor, respectively, for about 2 weeks, through an optical microscope.
FIG. 8 shows a photograph of the differentiated neural cell in FIG. 6 by immunocytochemistry; FIG. 8a shows a cell positively stained in NSE, FIG. 8b shows staining in NeuN, FIG. 8c shows staining in GFAP, and FIG. 8d shows staining in MAP-2.
FIG. 9 shows a photograph of a rat brain section about 2 weeks after a rat with induced ischemic disease received venous injection of about 3x10⁵ cells of the neural cells from the mesenchymal stem cell, through optical microscope; FIG. 9a shows a photograph of a rat brain before induction of local ischemic disease, FIG. 9b shows a photograph of a rat brain about 2 weeks after the venous injection of the neural cells after induction of local ischemic disease, and FIG. 9c shows an enlarged photograph of the local ischemic portion in FIG. 9b.

### Best Mode For Carrying Out the Invention

Hereinafter, the best mode of the present invention will be described in detail. However, it should be understood that these examples are provided only for illustration of the present invention, but not intended to limit the present invention in any manner.

### <Example 1> Isolation of mononuclear cells in bone marrow

About 10ml of bone marrow was obtained from the pelvises of healthy applicants and kept in glass tube comprising Heparin. About 30ml of phosphate buffered saline (PBS) was added to about 10ml of the bone marrow, about 20ml of the mixed solution was flowed slowly on about 10ml of Ficoll-PaqueTM plus (1.077g/ml, Amersham Pharmacia Biotech) solution and centrifuged on density gradient at 2,000rpm for about 20 minutes. The layer of a mononuclear cell was collected between an upper layer and the Ficoll-PaqueTM plus layer and centrifuged at 1,800rpm for about 5 minutes. Thus, the mononuclear cells were only obtained.

### <Example 2> Culture of the mononuclear cells

The mononuclear cells prepared in Example 1 were inoculated into culture flasks at about 1x10⁶ cells/cm² and after about 4 hours unanchored cells were removed by washing with a new basal medium. The new basal medium used was Wiliams' E medium (Gibco BRL) comprising 3.5M hydrocortisone (Sigma), fatty acid free bovine serum albumin (Gibco BRL), 50ng/ml linoleic acid (Sigma), 0.1M CuSO₄· 5H₂O (Sigma), 50pM ZnSO₄· 7H₂O (Sigma), 3ng/ml H₂· SeO₃ (Sigma), 1.05mg/ml NaHCO₃ (Sigma), 1.19mg/ml HEPES (Sigma), 100U/ml Penicillin (Gibco BRL), 10mg/ml Streptomycin (Gibco BRL), and 25g/ml Amphotericin (Gibco BRL).

### <Example 3> Differentiation of the mononuclear cells into neural cells without confluent culture

Example 3 confirmed whether the mononuclear cells cultured in Example 2 were differentiated into neural cells in a medium comprising about 10ng/ml of the epidermal growth factor (Gibco BRL) and about 20ng/ml of the hepatocyte growth factor (R&D systems) without confluent culture. Then the differentiation medium was changed twice per week.

When the mononuclear cells were cultured in the differentiation medium, the morphological change of the mononuclear cells was not detected after about 1 week. The neural cell colony appeared after about 4 weeks, and proliferated continuously. Neurons with long axons and short dendrites, and astrocytes with only short dendrites were observed after about 8 weeks. Further, from about 8 weeks, Example 3 was confirmed to proliferate maintaining same morphology even in a medium comprising only epidermal growth factor.

In contrast to culturing in the medium comprising the epidermal growth factor and the hepatocyte growth factor, the mononuclear cell was not differentiated into neural cells in a medium comprising only the epidermal growth factor, and was differentiated early and thus the mononuclear cell was not proliferated in a medium comprising only the hepatocyte growth factor.

Table 1 below shows the number of cells proliferated after the mononuclear cells were cultured in the medium comprising the epidermal growth factor and the hepatocyte growth factor for about 4 weeks without confluent culture, and Table 2 shows the proliferation conduct of the cells after the cells pretreated in Table 1 were cultured in a medium comprising only the epidermal growth factor or the hepatocyte growth factor for about 4 and about 8 weeks, respectively (the number of cells inoculated the beginning is about 1x10⁵).

### <Example 4> Differentiation of the mononuclear cells into neural cells after confluent culture

In Example 4, it was confirmed that the mononuclear cells cultured in Example 2 were differentiated into neural cells in a differentiation medium comprising about 10ng/ml of the epidermal growth factor (Gibco BRL) and about 20ng/ml of the hepatocyte growth factor (R&D systems) after confluent culture for about 24 hours. Then the differentiation medium was changed twice per week.

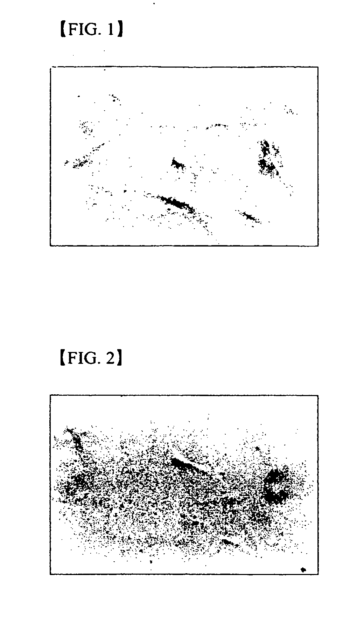

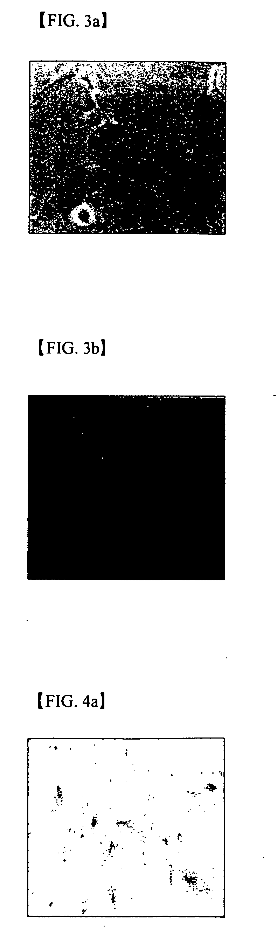

When the mononuclear cells were cultured in the differentiation medium, the neural cell colony started to appear after about 1 week, and proliferated continuously (refer to FIG. 1). When the mononuclear cells were cultured in the differentiation medium, neurons with long axons and short dendrites, and astrocytes with only short dendrites were observed after about 2 weeks (refer to FIGS. 2 and 3). Further, from about 2 weeks, Example 4 was confirmed to proliferate maintaining same morphology even in a medium comprising only the epidermal growth factor.

In contrast to culture in the medium comprising the epidermal growth factor and the hepatocyte growth factor, the mononuclear cells were not differentiated into neural cells in the medium comprising only the epidermal growth factor, and were differentiated early and so did not proliferate in the medium comprising only the hepatocyte growth factor.

Table 1 shows the number of cell proliferated after mononuclear cells were cultured in the medium comprising the epidermal growth factor and the hepatocyte growth factor for about 2 weeks after confluent culture for about 24 hours, and Table 2 shows the number of cells which conducted proliferation after cells were cultured in the medium comprising only the epidermal growth factor or the hepatocyte growth factor for about 4 and about 8 weeks, respectively, as pretreated (number of cell inoculated in the beginning is about 1x10⁵).

**[Table 1]**

| Pretreatment | Time (week) | Number of cells inoculated | Only HGF | Only EGF | EGF and HGF |
|---|---|---|---|---|---|
| Non-confluent culture | 4 | 7.5x10⁷ | Not proliferated | 1x10⁵ | 2.0x10⁵ |
| Confluent culture | 2 | 7.5x10⁷ | Not proliferated | 1.2x10⁵ | 1.8x10⁵ |

**[Table 2]**

| Pretreatment | Time (week) | Only HGF | Only EGF | EGF and HGF |
|---|---|---|---|---|
| Non-confluent culture | 4 | Not proliferated | 2x10⁵ | 2x10⁵ |
| | 8 | Not proliferated | 5x10⁵ | 1x10⁵ |
| Confluent culture | 4 | Not proliferated | 2x10⁵ | 1.7x10⁵ |
| | 8 | Not proliferated | 5.10⁵ | 1.3x10⁵ |

### <Example 5> Immunocytochemistry I

The cells differentiated in Examples 3 and 4 were adhered on the surface of a cover glass at about 1x10⁴ cells/cm². Subsequently, the cells were washed with 0.1M phosphate buffer for about 5 minutes, fixed with 0.1M phosphate buffer comprising 4% by weight paraformaldehyde for about 15 minutes, and washed with 0.1M PBS (phosphate buffered saline) for about 15 minutes twice. Then, the fixed cells were treated with 0.1M PBS comprising 0.1 % by weight bovine serum albumin (BSA) and 0.2% by weight triton X-100 for about 5 minutes, a first antibody was added to the fixed cells, and the fixed cells and the first antibody were reacted for about 16 hours. As the first antibody, anti-human neuron-specific enolase (NSE; Chemicon), anti-human NeuN (Chemicon), anti-human tubulin III (Sigma), anti-human glial fibrillary acidic protein (GFAP; Sigma), and anti-human MAP-2 (microtubule-associated protein-2) antibody were used. After reaction with the first antibody, the unbound first antibody was removed and the cells reacted with the first antibody were washed with 0.1M PBS comprising 0.5% by weight BSA for about 5 minutes twice. A second antibody was added to the cells reacted with the first antibody and the cells added with the second antibody were incubated for about 30 minutes. The incubated cells were then washed with 0.1M PBS comprising 0.5% by weight BSA for about 15 minutes twice. Vectastain Elite ABC kit (Vector Laboratory Inc.) comprising Avidin-biotin was used and reacted with the incubated cells for about 30 minutes. The reacted cells were washing with 0.1M PBS for about 5 minutes twice, and reacted with DAB (3,3'-diaminobenzidine tetrahydrochloride dehydrate, Sigma) as color-developing substrate for about 5 minutes. 0.1M PBS was added to the cells reacted with DAB for about 5 minutes in order to stop the reaction, and the cells quenching the reaction were washed with the 0.1M PBS for about 5 minutes twice. The reactant was dried and washed with distilled H₂O. The dried reactant was then dehydrated with distilled H₂O, 70%, 80%, 95% and 100% by weight ethanol in that order and fixed.

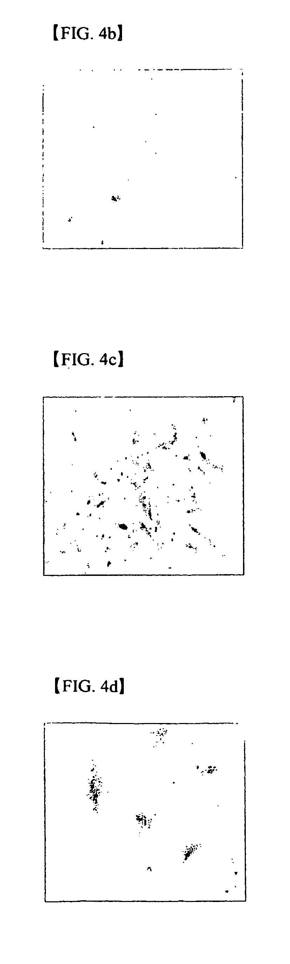

As a result of immunocytochemistry, FIG. 4 shows that the differentiated cells were stained positively in a neuron marker such as NeuN, NSE, MAP-2, and tubulin III and in an astrocyte marker such as GFAP, whereas the differentiated cells were stained negatively in a microglia marker such as OX-42. The result showed that the mononuclear cells were differentiated into neurons and astrocytes not only morphologically but also biochemically in the medium comprising the epidermal growth factor and the hepatocyte growth factor, however, the mononuclear cells were not differentiated into microglial cells.

The mononuclear cells were cultured in a medium comprising only the epidermal growth factor, a medium comprising the epidermal growth factor and the hepatocyte growth factor, and a medium comprising the epidermal growth factor and the hepatocyte growth factor after confluent culturing for about 24 hours, respectively. Subsequently, the differentiated cells were stained by immunocytochemistry to observe a ratio of the neurons (stained positively in NeuN, NSE, MAP-2 and tubulin III) and the astrocytes (stained positively in GFAP). The result is shown in Table 3.

**[Table 3]**

| | Time (week) | NSE | NeuN | GFAP | Map 2 | Negative cells |
|---|---|---|---|---|---|---|
| Only EGF | 4 | 0.9% | 0.8% | 1.2% | - | 89% |
| EGF + HGF | 2 | 10% | 25% | 4% | - | 70% |
| EGF + HGF | 4 | 56% | 75% | 24% | - | 20% |
| EGF + HGF After confluent culture for 24 hours | 2 | 62% | 88% | 31% | 11% | 10% |

As shown in Table 3, when the mononuclear cells were differentiated in the medium comprising the epidermal growth factor and the hepatocyte growth factor for about 2 weeks after confluent culture for about 24 hours, about 80% by count of the cells were differentiated into neural cells; about 70% by count of the neural cells are neurons and about 30% by count are astrocytes.

### <Example 6> Isolation and culture of the mesenchymal stem cells

To confirm whether the mesenchymal cells were differentiated into neural cells, the mesenchymal cells were isolated from the bone marrow-derived mononuclear cells and an experimentation regarding differentiative potential was carried out.

The mononuclear cells cultured in Example 2 were inoculated in a culture flask at about 1x10⁶ cells/cm² and incubated at a temperature of about 37°C in CO₂ incubator. As a medium, Gibco BRL (DMEM) of low glucose concentration with fetal bovine serum (FBS) being added to the medium at about 10% by weight. After culturing of the mononuclear cells for about 1 to about 2 weeks, the mononuclear cells were fully proliferated for a subculture, and the mononuclear cells continued to proliferate after 20 subculturings.

The mononuclear cell group derived from the bone marrow contains leukocytes, lymphocytes, osteoblasts, chondroblasts, muscle cells, fibroblasts, fat cells and stem cells. The stem cells may be differentiated into the leukocytes, lymphocytes, osteoblasts, chondroblasts, muscle cells, fibroblasts, fat cells, etc. The stem cells indicate hematopoietic stem cells and mesenchymal stem cells. The hematopoietic stem cells of forming blood cells such as erythrocytes, leucocytes and lymphocytes are not continually proliferated in a normal culture medium, and are differentiated into mature cells such that the continually proliferating cells are the mesenchymal stem cells.

For confirmation, Example 6 examined whether the above-obtained cells have the differentiative potential of the mesenchymal stem cells by adding kinds of cytokines. That is, the possibility of differentiation into connective tissues such as osteoblasts, chondroblasts and fat cells was tested.

For differentiation into osteoblasts, the above-obtained cells with added kinds of cytokines were incubated with 100mM dexamethasone, 10mM glycerol phosphate, 50nM ascorbate-2-phosphate, and 10% by weight fetal bovine serum (FBS). For differentiation into chondroblasts, the incubated cells were centrifuged at 1500rpm for about 10 minutes such that the incubated cells formed a pellet and then the pellet was added with 100nM dexamethasone and 10ng/ml TGF-3 in serum-free state. For differentiation into fat cells, the above-obtained cells with added kinds of cytokines were incubated with 0.5mM 1-methyl-3-isobutylxanthine, 1mM dexamethasone, 10g/ml insulin, and 10nM indomethacine (Pittenger et al., Science 284:143-147, 1999). Differentiation into each cell (type) was confirmed by alkaline phosphatase staining in osteoblasts (Jaiswal et al., J. Cell Biochem. 64(2) 143-147, 1999), type II collagen staining RT-PCR by Toluidine blue in chondroblasts (Mackay et al., Tissue Eng. 4(4): 415-428, 1998), and oil red O staining in fat cells, respectively.

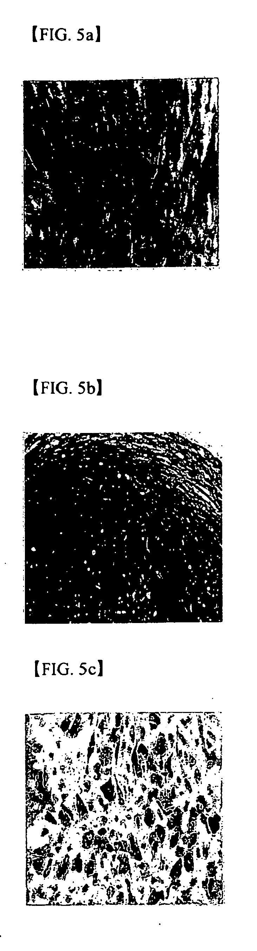

As a result, as shown in FIGS. 5a, 5b, and 5c, all were positively stained. The mesenchymal stem cells cultured ex vivo still had differentiative potential into connective tissues such as osteoblasts, chondroblasts and fat cells.

### <Example 7> Differentiation of the mesenchymal stem cells into neural cells without confluent culture

To confirm whether the mesenchymal stem cell isolated in Example 6 differentiated into neural cells, the mesenchymal stem cells cultured ex vivo in DMEM of low glucose concentration, the DMEM comprising 10% by weight FBS, were cultured in the differentiation medium comprising about 10ng/ml of the epidermal growth factor and about 20ng/ml of the hepatocyte growth factor according to the method of Example 3 for 8 weeks without confluent culturing.

The result was same as neural cells differentiated from the bone marrow-derived mononuclear stem cells; a morphological change was not detected after about 1 week, and the neural cell colony appeared after about 4 weeks, and continually proliferated after about 5 weeks. After about 8 weeks, Example 7 confirmed to proliferate in same morphology even in case of culturing in the medium comprising only the epidermal growth factor.

### <Example 8> Differentiation of the mesenchymal stem cells into neural cells after confluent culture

To confirm whether the mesenchymal stem cells isolated in Example 6 differentiated into neural cells, the mesenchymal stem cells, cultured ex vivo in DMEM of low glucose concentration, the DMEM comprising 10% by weight FBS, were cultured in the differentiation medium comprising about 10ng/ml of the epidermal growth factor and about 20ng/ml of the hepatocyte growth factor according to the method of Example 4 for about 2 weeks after confluent culture for about 24 hours.

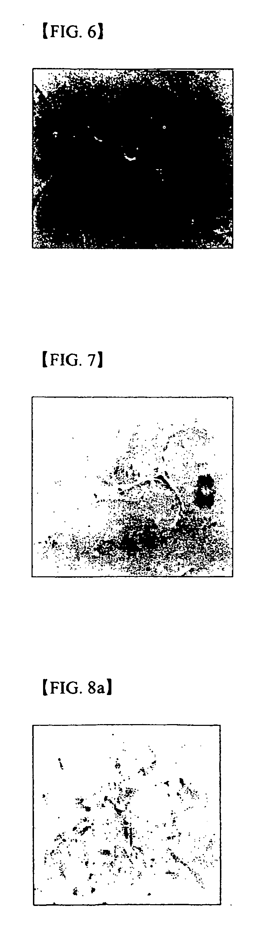

The result was same as neural cells differentiated from the bone marrow-derived mononuclear stem cells; a morphological change was not detected after about 1 week, and the cells continually proliferated after about 2 weeks (refer to FIGS. 6 and 7). After about 2 weeks, Example 8 confirmed to proliferate in the same morphology even in case of culturing in the medium comprising only the epidermal growth factor.

### <Example 9> Immunocytochemistry II

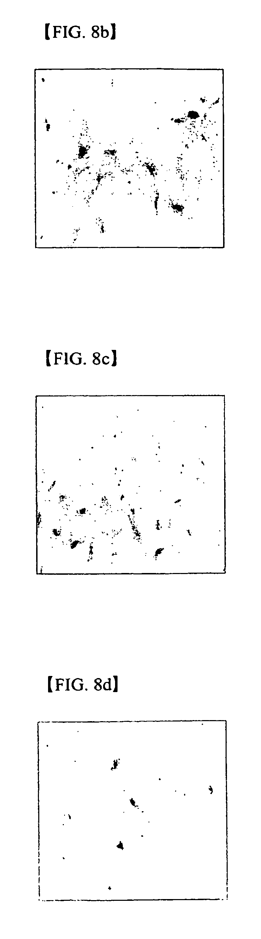

The cells differentiated in Examples 7 and 8 were stained by immunocytochemistry as in the method of Example 5. The result was same as neural cells differentiated from the bone marrow-derived mononuclear stem cells; the neural cells differentiated from the mesenchymal stem cell were stained positively in a neuron marker such as NeuN, NSE, MAP-2, and tubulin III and in an astrocyte marker such as GFAP (Figure 8a, 8b, 8c, and 8d). In the case of performing a confluent culture as pre-treatment, the result showed that mesenchymal stem cells were differentiated into neurons and astrocytes, and that the differentiation rate was faster by 2 times and the efficiency of differentiation was increased by 80% to 90% than in the case without performing the confluent culture as pre-treatment.

### <Example 10> (for reference purposes)

To confirm whether the function of the neural cells differentiated from the mesenchymal stem cells was equal to the function of human neural cells, Example 10 was carried out by obtaining and fixing a damaged portion wherein the neural cells were implanted into said damaged portion of the animal.

To induce ischemic brain disease, known as common neurodegenerative disease in human, in a brain of an experimental rat, the middle cerebral artery was cut, was fastened for 1 hour and loosened again such that the brain of the experimental rat was provided with local ischemic disease in cerebrum cortex. And then the neural cells prepared by the method of Example 8 were venous-injected at about 3x10⁵ cells. Then the injected neural cells were stained, to distinguish them from other cells, with LacZ.

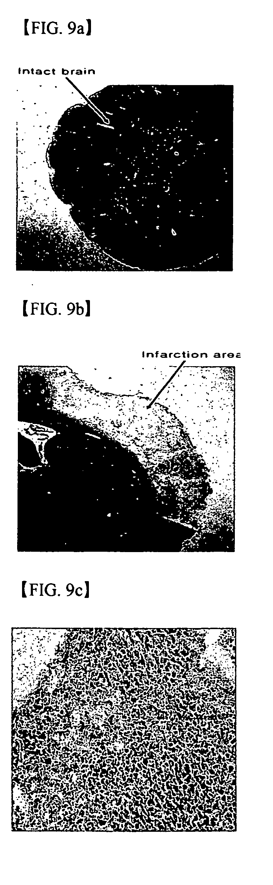

1 week after the venous injection, the brain of the experimental rat was cut and observed through optical microscope. The result is shown in FIG. 9; FIG. 9a shows a photograph of the rat brain before induction of local ischemic disease, FIG. 9b shows a photograph of the rat brain 2 weeks after the neural cells were venous-injected after induction of local ischemic disease, and FIG. 9c shows an enlarged photograph of the local ischemic portion in FIG. 9b.

As shown in FIGS. 9a and 9b, a difference between normal and ischemic brain was clearly confirmed. Especially, it was detected that cells stained weakly in FIGS. 9b and 9c were stained strongly with LacZ. Accordingly, the neural cells were selectively arrived at the damaged portion. That is, the mesenchymal stem cells were normally differentiated into neural cells and the neural cells were located in an appropriate portion. Therefore, the neural cells differentiated according to the method of the present invention are useful for neurodegenerative disease.

### Industrial applicability

As mentioned above, the present invention provides a method of differentiating and proliferating a mesenchymal stem cell or a mononuclear cell into a neural cell consisting of a neuron and an astrocyte more effective, in terms of time, efficiency and maturity, and a sufficient amount of neural cells to produce a pharmaceutical composition for treating neurodegenerative disease. The method is safe and has little problem with clinical application due to using a natural compound in a cell. The method has effects regarding little immunological response and supplies a sufficient amount of neural cells due to the obtention of a large quantity of neural cells from a patient's bone marrow.

## Claims

1. A method of differentiating and proliferating a mesenchymal stem cell into a neuron or astrocyte, comprising the two steps of;
(1) Confluent culturing the mesenchymal stem cell as a pretreatment, and then
(2) Culturing said mesenchymal stem cell in a medium comprising an epidermal growth factor and a hepatocyte growth factor.

2. The method of claim 1, wherein the mesenchymal stem cell is cultured for more than 1 week in a medium comprising about 1 to about 10,000ng/ml of the epidermal growth factor and about 1 to about 10,000ng/ml of the hepatocyte growth factor after confluent culturing of said mesenchymal stem cell for about 1 to about 50 hours.

3. The method of claim 1, wherein the mesenchymal stem cell is cultured for more than 1 week in a medium comprising about 10ng/ml of the epidermal growth factor and about 20ng/ml of the hepatocyte growth factor after confluent culturing of said mesenchymal stem cell for about 24 hours.

4. The method of any one of claims 1 to 3, wherein the mesenchymal stem cell is cultured for about 2 weeks in the medium comprising the epidermal growth factor and the hepatocyte growth factor and then the medium comprising the epidermal growth factor and the hepatocyte growth factor is changed with a medium comprising only the epidermal growth factor.

5. The method of claim 4, wherein the mesenchymal stem cell is a mononuclear cell obtained from bone marrow.

## Patentansprüche

1. Verfahren zum Differenzieren und Proliferieren einer mesenchymalen Stammzelle zu einem Neuron oder einem Astrocyten, umfassend die zwei Stufen des:
(1) konfluenten Kultivierens der mesenchymalen Stammzelle als eine Vorbehandlung und dann
(2) Kultivieren der mesenchymalen Stammzelle in einem Medium, umfassend einen epidermalen Wachstumsfaktor und einen Hepatocytenwachstumsfaktor.

2. Verfahren nach Anspruch 1, worin die mesenchymale Stammzelle für mehr als eine Woche in einem Medium, umfassend etwa 1 bis etwa 10.000 ng/ml des epidermalen Wachstumsfaktors und etwa 1 bis etwa 10.000 ng/ml des Hepatocytenwachstumsfaktors, nach konfluentem Kultivieren der mesenchymalen Stammzelle für etwa 1 bis etwa 50 Stunden kultiviert wird.

3. Verfahren nach Anspruch 1, worin die mesenchymale Stammzelle für mehr als 1 Woche in einem Medium, umfassend etwa 10 ng/ml des epidermalen Wachstumsfaktors und etwa 20 ng/ml des Hepatocytenwachstumsfaktors, nach konfluentem Kultivieren der mesenchymalen Stammzelle für etwa 24 Stunden kultiviert wird.

4. Verfahren nach einem der Ansprüche 1 bis 3, worin die mesenchymale Stammzelle für etwa 2 Wochen in dem Medium, umfassend den epidermalen Wachstumsfaktor und den Hepatocytenwachstumsfaktor, kultiviert wird und dann das Medium, umfassend den epidermalen Wachstumsfaktor und den Hepatocytenwachstumsfaktor, durch ein Medium ausgetauscht wird, das nur den epidermalen Wachstumsfaktor umfasst.

5. Verfahren nach Anspruch 4, worin die mesenchymale Stammzelle eine aus Knochenmark erhaltene mononukleäre Zelle ist.

## Revendications

1. Procédé de différenciation et de prolifération d'une cellule souche mésenchymateuse dans un neurone ou un astrocyte, comprenant les deux étapes consistant à :
(1) cultiver jusqu'à la confluence la cellule souche mésenchymateuse en tant que prétraitement, et ensuite
(2) cultiver ladite cellule souche mésenchymateuse dans un milieu comprenant un facteur de croissance des cellules épidermiques et un facteur de croissance des hépatocytes.

2. Procédé selon la revendication 1, dans lequel la cellule souche mésenchymateuse est cultivée pendant plus de 1 semaine dans un milieu comprenant environ 1 à environ 10000 ng/ml du facteur de croissance des cellules épidermiques et environ 1 à 10000 ng/ml du facteur de croissance des hépatocytes après la culture jusqu'à la confluence de ladite cellule souche mésenchymateuse pendant environ 1 à environ 50 heures.

3. Procédé selon la revendication 1, dans lequel la cellule souche mésenchymateuse est cultivée pendant plus de 1 semaine dans un milieu comprenant environ 10 ng/ml du facteur de croissance des cellules épidermiques et environ 20 ng/ml du facteur de croissance des hépatocytes après la culture jusqu'à la confluence de ladite cellule souche mésenchymateuse pendant environ 24 heures.

4. Procédé selon l'une quelconque des revendications 1 à 3, dans lequel la cellule souche mésenchymateuse est cultivée pendant environ 2 semaines dans le milieu comprenant le facteur de croissance des cellules épidermiques et le facteur de croissance des hépatocytes et ensuite le milieu comprenant le facteur de croissance des cellules épidermiques et le facteur de croissance des hépatocytes est changé par un milieu comprenant uniquement le facteur de croissance des cellules épidermiques.

5. Procédé selon la revendication 4, dans lequel la cellule souche mésenchymateuse est une cellule mononucléée obtenue à partir de la moelle osseuse.
